**Europäisches Patentamt**

**European Patent Office**

**Office européen des brevets**

(19)

(11) Veröffentlichungsnummer : **0 119 286**

**B1**

(12)

# EUROPÄISCHE PATENTSCHRIFT

(45) Veröffentlichungstag der Patentschrift :
23.12.87

(51) Int. Cl.⁴ : **A 61 M 5/30**

(21) Anmeldenummer : **83102688.5**

(22) Anmeldetag : **18.03.83**

(54) **Druckmittelbetätigte Injektionspistole.**

(43) Veröffentlichungstag der Anmeldung :
**26.09.84 Patentblatt 84/39**

(45) Bekanntmachung des Hinweises auf die Patenterteilung : **23.12.87 Patentblatt 87/52**

(84) Benannte Vertragsstaaten :
**DE FR GB NL**

(56) Entgegenhaltungen :
**DE-A- 2 706 217**
**DE-B- 1 944 006**
**US-A- 2 729 198**
**US-A- 3 087 160**
**US-A- 3 278 104**

(73) Patentinhaber : **Internationales Forschungsinstitut für Reproduktionsmedizin und -biologie**
**Kaiser-Wilhelm-Ring 22**
**D-4000 Düsseldorf (DE)**

(72) Erfinder : **Kurz, Karl-Heinz, Dr.**
**Kaiser-Wilhelm-Ring 22**
**D-4000 Düsseldorf 11 (DE)**
Erfinder : **Gutierrez, Javier, Dipl.-Ing.**
**Blumenstrasse 5**
**D-4005 Meerbusch 1 (DE)**

(74) Vertreter : **Tackenberg, Karl, Dipl.-Ing.**
**Birkenweiher 15**
**D-5650 Solingen (DE)**

EP 0 119 286 B1

## Beschreibung

Die Erfindung betrifft eine druckmittelbetätigte, nadellose Injektionspistole, mit einem in einem Zylinder angeordneten, mit einem Pumpenkolben zusammenwirkenden Arbeitskolben, einem mittels eines an dem Körper der Injektionspistole angelenkten Handhebels über einen Stößel steuerbaren, federbelasteten Einlaßventil, über das der Zylinder des Arbeitskolbens mit einer Druckmittelquelle verbindbar ist, und einem lösbaren Gesperre, durch das der Arbeitskolben bei Erreichen eines vorbestimmten Druckes im Zylinder auslösbar ist (Siehe DE-B-1 944 066).

Bei den bekannten Injektionspistolen läßt sich nicht ausschließen, daß das zu injizierende Mittel infolge Druckabsenkung während des Arbeitshubes des Arbeitskolbens mit zu niedrigem Druck injiziert wird. Dies hat zur Folge, daß das Mittel nicht zerstäubt wird, sondern in einem Strahl auf die Haut auftrifft, was Hautverletzungen verursachen kann. Es sind ferner die herkömmlichen Injektionspistolen (vgl. beispielsweise DE-B-1 944 066 und DE-A-27 06 217) relativ kompliziert im Aufbau, wodurch das Zerlegen der Injektionspistole zum Zwecke des Reinigens und Sterilisierens der Einzelteile erschwert ist.

Der Erfindung liegt daher die Aufgabe zugrunde, eine druckmittelbetätigte, nadellose Injektionspistole der eingangs beschriebenen Gattung im Aufbau wesentlich zu vereinfachen, wobei das Einlaßventil für das Druckmittel sowie das Riegelglied für den Arbeitskolben mit einem einzigen Handhebel betätigt werden und der Arbeitskolben während des Ausführens eines Arbeitshubes unter konstantem Druck des Druckmittels steht.

Die Lösung der Aufgabe besteht erfindungsgemäß darin, daß

a) der Handhebel an seinem vorderen Ende einen Haken bildet und mit einer äußeren Krümmung an dem einen bogenförmig verlaufenden Ende eines in einer Ausnehmung der Wandung des Körpers der Injektionspistole geführten Schiebers anliegt, der mit seinem anderen Ende dem einen Ende des federbelasteten Stößels des Einlaßventiles zugekehrt ist,

b) der Handhebel mit seinem Haken in einer länglichen Ausnehmung am Umfang eines in dem Körper der Injektionspistole verschiebbar gelagerten federbelasteten Sperriegels liegt, der in der Riegellage dem Arbeitskolben vorgeordnet ist.

Die Auslösung des Arbeitskolbens erfolgt erst dann, wenn der Zylindervorraum mit dem Druckmittel gefüllt ist, wobei während des Ausführens eines Arbeitshubes durch den Arbeitskolben Druckmittel aus der Druckmittelquelle nachfließt, da auch während des Ausführens eines Arbeitshubes das Einlaßventil für das Druckmittel geöffnet bleibt. Der Arbeitskolben steht hierdurch während des Ausführens eines Arbeitshubes unter dem konstanten Druck des Druckmittels, wodurch ein einwandfreies Zerstäuben des zu injizierenden Mittels gewährleistet ist.

Eine vorteilhafte Weiterbildung der erfindungsgemäßen Injektionspistole besteht darin, daß der stößel des Einlaßventiles als Hohlrohr ausgebildet ist und mit seinem einen Ende mit einer Nut des Schiebers in Wirkverbindung steht, und daß das Einlaßventil als ein das Hohlrohr des Stößels schließender Ventilkörper ausgebildet ist.

Das nach Ausführen eines Arbeitshubes durch den Arbeitskolben in dem Zylindervorraum noch vorhandene Druckmittel strömt nach Lösen des Handhebels durch den Stößel und durch die Nut des Schiebers hindurch ins Freie, wodurch die Anordnung eines gesonderten Arbeitskanales entfällt.

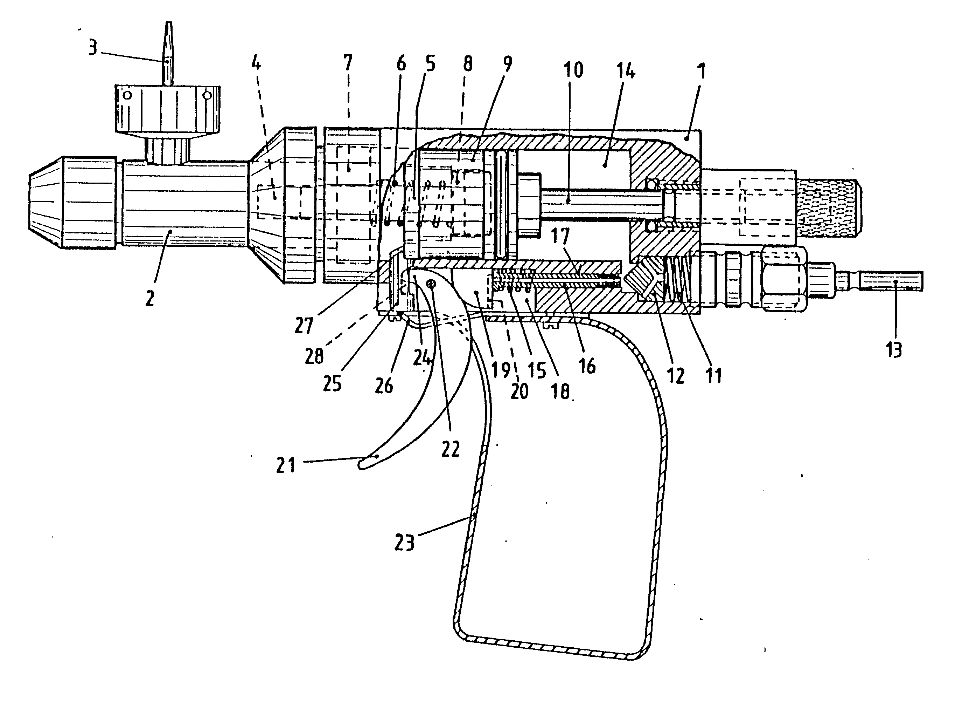

Die Erfindung ist nachstehend in einem Ausführungsbeispiel anhand der Zeichnung näher erläutert.

Mit 1 ist der Körper der Injektionspistole bezeichnet, in derem Kopfteil 2 eine Ausspritzkammer untergebracht ist, die mit einem flüssigen Medikament aus einem Impfstoffbehälter gefüllt wird. Der Impfstoffbehälter ist auf eine außen am Körper 1 angeordnete Stichverbindung 3 aufsteckbar, die die Verbindung zu einer mit einem Rückschlagventil versehenen Leitung herstellt, durch die hindurch der Impfstoff in die Ausspritzkammer gelangt. Mit 4 ist ein Pumpenkolben bezeichnet, mittels dessen in bekannter Weise das Medikament durch eine Zerstäuberdüse unter hohem Druck in die Haut des Patienten gespritzt wird. Der Pumpenkolben 4 weist rückseitig eine Stange 5 auf, auf der eine Schraubenfeder 6 angeordnet ist. Die Schraubenfeder 6 ist unter Vorspannung mit ihrem einen Ende an einem ringförmigen Vorsprung 7 im Innern des Körpers 1 und mit ihrem anderen Ende an einem auf dem freien Ende der Stange 5 fest angeordneten Anschlag 8 abgestützt. Mit 9 ist der topfförmige Arbeitskolben bezeichnet, bis in den die Stange 5 mit dem Anschlag 8 ragt. Mit 10 ist die Kolbenstange des Arbeitskolbens 9 bezeichnet, die das hintere Ende des Körpers 1 durchsetzt.

In der Wandung des Körpers 1 ist ein mittels einer Feder 11 belastetes Einlaßventil 12 für ein Druckmittel angeordnet, das als ein das Hohlrohr eines Stößels 16 schließender Ventilkörper ausgebildet ist. Bei geöffnetem Einlaßventil 12 strömt das Druckmittel aus einem Vorratsbehälter durch eine Rohrleitung 13 hindurch in den Zylindervorraum 14. Die Betätigung des Einlaßventiles 12 erfolgt mittels des durch eine vorgespannte Schraubenfeder 15 belasteten Stößels 16, der in einer zylindrischen Ausnehmung 17 des Körpers 1 gelagert ist. In einer sich an die Ausnehmung 17 anschließenden Ausnehmung 18 des Körpers 1 ist ein mit seinem einen Ende dem einen Ende des Stößels 16 vorgelagerter Schieber 19 geführt, der an seinem dem Stößel 16 zugekehrten Ende eine Nut 20 aufweist. Die Schraubenfeder 15 stützt sich mit ihrem einen Ende an der einen endseitigen Begrenzung der Ausnehmung 18 und mit ihrem anderen Ende an einer Verbreiterung des Mantels des stößels 16 ab. Der Schieber 19 ist an seinem anderen Ende bogenförmig gestaltet. Mit 21 ist ein bei 22 angelenkter Handhebel bezeichnet, dem eine Handhabe 23 zugeordnet ist. Der Handhebel 21 liegt mit seinem gekrümmten Rücken an dem bogenförmig gestalteten Ende des Schiebers 19 an. Er bildet an seinem vorderen Ende einen Haken 24. Mit 25 ist ein in der Wandung des Körpers 1 verschiebbar gelagerter, bolzenförmiger Sperriegel bezeichnet, der unter der Druckwirkung einer blattförmigen Feder 26 steht. Die Feder 26 ist zusammen mit der Handhabe 23 außen am Körper 1 befestigt. Der Sperriegel 25 weist an seinem der Feder 26 abgekehrten Ende einen Absatz 27 auf, mit dem er an dem Arbeitskolben 9 stirnseitig und am Umfang anliegt. Er besitzt am Umfang eine Ausnehmung 28, in der der Handhebel 21 mit dem Haken 24 liegt.

Der Handhebel 21 übt beim Anziehen Druck auf das benachbarte Ende des Schiebers 19 aus, der sich dabei verschiebt und den Stößel 16 entgegen der Wirkung der Feder 11 so weit mitnimmt, bis das Einlaßventil 12 von seinem Sitz abgehoben ist. Hiernach strömt das Druckmittel in den Zylindervorraum 14 und baut sich hier zu einem vorbestimmten Druck auf. Bei weiterem Anziehen des Handhebels 21 zieht dieser den Sperriegel 25 entgegen der Wirkung der Feder 26 so weit an, bis der Arbeitskolben 9 freigegeben ist. Es bleibt dabei das Einlaßventil 12 unter dem Druck des Stößels 16 geöffnet. Der Arbeitskolben 9 vollführt hiernach durch den Druck des in dem Zylindervorraum 14 eingeschlossenen und nachströmenden Druckmittels einen Arbeitshub, indem er durch Druck auf die Stange 5 des Pumpenkolbens 4 den Pumpenkolben 4 betätigt. Bei nachlassendem Zug an dem Handhebel 21 wird der Stößel 16 zusammen mit dem Schieber 19 und dem Handhebel 21 durch die Wirkung der vorher gespannten und sich nunmehr entspannenden Schraubenfeder 15 des Stößels 16 in die Ausgangslage bewegt. Dabei rückt das Einlaßventil 12 durch die Wirkung der Feder 11 wieder in die Schließlage, wodurch die Zufuhr des Druckmittels unterbrochen ist. Das dem Einlaßventil 12 zugekehrte, zuvor geschlossene Ende des Stößels 16 ist nunmehr geöffnet, wodurch im Zylindervorraum 14 vorhandenes Druckmittel durch den Stößel 16 hindurch in die Nut 20 des Schiebers 19 und von dort ins Freie abgeleitet wird.

Es wird ferner der Arbeitskolben 9 durch die Wirkung der vorher gespannten und sich nunmehr entspannenden Schraubenfeder 6 zusammen mit dem Pumpenkolben 4 in die Ausgangslage bewegt, in der der durch den Handhebel 21 freigegebene Sperriegel 25 durch die Wirkung der vorher gespannten und sich nunmehr entspannenden Feder 26 wieder in die Riegellage rückt.

**Patentansprüche**

1. Druckmittelbetätigte, nadellose Injektionspistole, mit einem in einem Zylinder angeordneten, mit einem Pumpenkolben zusammenwirkenden Arbeitskolben, einem mittels eines an dem Körper der Injektionspistole angelenkten Handhebels über einen Stößel steuerbaren, federbelasteten Einlaßventil, über das der Zylinder des Arbeitskolbens mit einer Druckmittelquelle verbindbar ist, und einem lösbaren Gesperre, durch das der Arbeitskolben bei Erreichen eines vorbestimmten Druckes im Zylinder auslösbar ist, dadurch gekennzeichnet, daß

a) der Handhebel (21) an seinem vorderen Ende einen Haken (24) bildet und mit einer äußeren Krümmung an dem einen bogenförmig verlaufenden Ende eines in der Ausnehmung (18) der Wandung des Körpers (1) der Injektionspistole geführten Schiebers (19) anliegt, der mit seinem anderen Ende dem einen Ende des Stößels (16) des Einlaßventiles (12) zugekehrt ist,

b) der Handhebel (21) mit seinem Haken (24) in einer länglichen Ausnehmung (28) am Umfang eines in dem Körper (1) der Injektionspistole verschiebbar gelagerten, federbelasteten Sperriegels (25) liegt, der in der Riegellage dem Arbeitskolben (9) vorgeordnet ist.

2. Druckmittelbetätigte, nadellose Injektionspistole nach Anspruch 1, dadurch gekennzeichnet, daß der Stößel (16) des Einlaßventiles (12) als Hohlrohr ausgebildet ist und mit seinem einen Ende mit einer Nut (20) des Schiebers (19) in Wirkverbindung steht, und daß das Einlaßventil (12) als ein das Hohlrohr des Stößels (16) schließender Ventilkörper ausgebildet ist.

**Claims**

1. Pressure-powered needleless injection pistol having a working piston arranged in a cylinder and cooperating with a pump piston, a spring-loaded inlet valve which is controllable via a ram by a hand lever pivotally attached to the body of the injection pistol, by means of which inlet valve the cylinder of the working piston can be connected to a pressuremedium source, and a releasable locking mechanism by means of which the working piston is released upon reaching a predetermined pressure in the cylinder, characterised in that

a) the hand lever (21) forms at its front end a hook (24) and engages with an outer curvature the arcuately extending end of a slide (19) guided in the recess (18) in the wall of the body (1) of the injection pistol, said slide facing with its other end one end of the ram (16) of the inlet valve (12),

b) the hand lever (21) is located with its hook (24) in a longitudinal recess (28) at the circumference of a spring-loaded locking bolt (25) which is slidably mounted in the body (1) of the injection pistol and, in the locking position, is arranged upstream of the working piston (9).

2. Pressure-powered needleless injection pistol as claimed in claim 1, characterised in that the ram (16) of the inlet valve (12) is formed as a hollow tube and is in operational connection with one of its ends with a groove (20) of the slide (19), and that the inlet valve (12) is formed as a valve body closing the hollow tube of the ram (16).

**Revendications**

1. Pistolet à injections sans pointeau actionné sous pression, avec un piston de travail disposé dans un cylindre et co-agissant avec un piston de pompe, une soupape d'admission chargée par ressort et commandée, par l'intermédiaire d'un poussoir, au moyen d'un levier à main articulé au corps du pistolet à injections, soupape à travers laquelle le cylindre du piston de travail est reliable à une source de pression, et avec un encliquetage déblocable susceptible de déclencher le piston de travail quand une pression prédéterminée est atteinte dans le cylindre, pistolet à injections caractérisé par ce que

a) le levier à main (21) forme, à son extrémité avant, un crochet (24) et s'appuie avec une courbure extérieure sur l'extrémité arquée d'une coulisse (19) guidée dans l'évidement (18) de la paroi du corps (1) du pistolet à injections et dirigée, avec son autre extrémité, vers l'une des extrémités du poussoir (16) de la soupape d'admission (12),

b) le levier à main (21) loge avec son crochet (24) dans un évidement oblong (28) au pourtour d'un verrou de sûreté (25) chargé par ressort et pouvant se déplacer dans le corps (1) du pistolet à injections et qui, en position de verrouillage, se trouve placé devant le piston de travail (9).

2. Pistolet à injections sans pointeau actionné sous pression suivant revendication 1, caractérisé par ce que le poussoir (16) de la soupape d'admission (12) est conçu comme tube creux et se trouve, avec l'une de ses extrémités, en jonction effective avec une rainure (20) de la coulisse (19), et que la soupape d'admission (12) est conçue comme un corps de soupape obturant le tube creux du poussoir (16).